(19) Europäisches Patentamt
European Patent Office
Office européen des brevets

(11) Veröffentlichungsnummer: **0 018 016**
**B1**

(12) # EUROPÄISCHE PATENTSCHRIFT

(45) Veröffentlichungstag der Patentschrift: **10.08.83**

(51) Int. Cl.³: **C 07 D 237/22,** A 61 K 31/50

(21) Anmeldenummer: **80102190.8**

(22) Anmeldetag: **23.04.80**

(54) **3-(3'-Amino-2'-hydroxy-n-prop-1'-oxy)-6-isopropylidenhydrazinopyridazine, Verfahren zu ihrer Herstellung und diese Verbindungen enthaltende Arzneimittel.**

(30) Priorität: **23.04.79 IT 2203579**

(43) Veröffentlichungstag der Anmeldung: **29.10.80 Patentblatt 80/22**

(45) Bekanntmachung des Hinweises auf die Patenterteilung: **10.08.83 Patentblatt 83/32**

(84) Benannte Vertragsstaaten: **AT BE CH DE FR GB IT LI NL**

(56) Entgegenhaltungen:

**BE-A-830 158**
**DE-A-2 154 245**
**DE-A-2 406 930**
**DE-A-2 556 918**

**Die Akte enthält technische Angaben, die nach dem Eingang der Anmeldung eingereicht wurden und die nicht in dieser Patentschrift enthalten sind.**

(73) Patentinhaber: **I. S. F. Società per Azioni, Via Leonardo da Vinci, 1, I-20090 Trezzano s/N (Milano) (IT)**

(72) Erfinder: **Dorigotti, Luciano, v. Benevento, 1, Milano (IT)**
Erfinder: **Gaviraghi, Giovanni, v. Madonnina, 11, Agrate Brianza (Milano) (IT)**
Erfinder: **Pinza, Mario, v. Prov. per Cesano Boscone, Corsico (Milano) (IT)**
Erfinder: **Pifferi, Giorgio, v. Carlone, 8, Milano (IT)**
Erfinder: **Semeraro, Claudio, v. Allende, 2, Bresso (Milano) (IT)**

(74) Vertreter: **Beszédes, Stephan G., Dr. et al, Am Heideweg 2 Postfach 1168, D-8060 Dachau (DE)**

**EP 0 018 016 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 3-(3'-Amino-2'-hydroxy-n-prop-1'-oxy)-6-isopropylidenhydrazinopyridazine, Verfahren zu ihrer Herstellung und diese Verbindungen enthaltende Arzneimittel

Die Erfindung betrifft neue 3-(3'-Amino-2'-hydroxy-n-prop-1'-oxy)-6-isopropylidenhydrazinopyridazine, ein Verfahren zu ihrer Herstellung und diese Verbindungen enthaltende Arzneimittel, insbesondere solche mit blutdrucksenkenden und gefässerweiternden Wirkungen.

Aus der DE-A-2 406 930 sind substituierte 3-Amino-2-hydroxy-n-prop-1-oxyheterocyclen, wie 3-Amino-2-hydroxy-n-prop-1-oxypyridazine, bei welchen die 3-Aminogruppe als Substituenten auch eine, gegebenenfalls durch eine Phenylgruppe substituierte, niedere Isoalkylgruppe oder tert.-Alkylgruppe aufweisen kann, bekannt. Der andere Substituent des heterocyclischen Ringes, wie Pyridazinringes, kann jedoch keine Alkylidenhydrazinogruppe und nicht einmal eine Hydrazinogruppe sein. Als pharmakologische Wirkungen dieser Verbindungen sind ihre β-rezeptorblockierenden und stimulierenden Wirkungen angegeben, wobei speziell für 3-Amino-2-hydroxy-n-prop-1-oxypyridazine eine pharmakologische Wirkung nicht ausdrücklich genannt ist. Von einer gefässerweiternden Wirkung ist in der ganzen Druckschrift überhaupt keine Rede. Auch ist es aus der DE-A-2 406 930 bekannt, 3-Halogen-2-hydroxy-n-prop-1-oxyheterocyclen mit Aminen zu substituierten 3-Amino-2-hydroxy-n-prop-1-oxyheterocyclen umzusetzen.

Ferner sind in der BE-A-830 158 sowie DE-A-2 556 918 3-Phenyl-6-hydrazinopyridazine, welche also am Pyridazinring zwingend einen Phenylrest haben, beschrieben. Sie weisen am Pyridazinring keinen 3-Amino-2-hydroxy-n-prop-1-oxysubstituenten auf. Als pharmakologische Wirkungen dieser Verbindungen sind ihre β-rezeptorenblockierenden, blutdrucksenkenden und gefässerweiternden Wirkungen genannt, wobei angegeben ist, dass sie keine Tachykardie hervorrufen. Auch ist es aus der DE-A-2 556 918 bekannt, Chlorpyridazinderivate mit Hydrazin und anschliessend mit Carbonylderivaten umzusetzen.

Weiterhin sind aus der DE-A-2 154 245 Pyridazinderivate mit einer substituierten Aminogruppe in der 6-Stellung und einer Hydrazino- oder Benzylidenhydrazinogruppe in der 3-Stellung bekannt. Diese Verbindungen haben eine blutdrucksenkende Wirkung.

Der Erfindung liegt die Aufgabe zugrunde, überlegene pharmakologische Wirkungen aufweisende neue 3-Alkoxy-6-isopropylidenhydrazinopyridazine, ein Verfahren zur Herstellung derselben und diese Verbindungen enthaltende Arzneimittel zu schaffen.

Das Obige wurde überraschenderweise durch die Erfindung erreicht.

Gegenstand der Erfindung sind 3-(3'-Amino-2'-hydroxy-n-prop-1'-oxy)-6-isopropylidenhydrazinopyridazine der allgemeinen Formel I

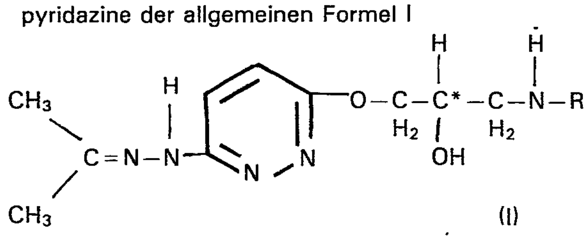

(I)

worin

R für einen Alkylrest mit 1 bis 4 Kohlenstoffatomen steht, der gegebenenfalls durch einen Phenylrest substituiert ist, welcher noch 1 bis 3 Methoxyreste tragen kann, und

das Sternchen (*) ein die Existenz von 2 optisch aktiven Formen herbeiführendes Asymmetriezentrum des Moleküls bedeutet,

in Form der getrennten optisch aktiven Isomere oder von Mischungen derselben sowie ihre Salze mit pharmazeutisch brauchbaren Säuren.

Bevorzugte Salze der 3-(3'-Amino-2'-hydroxy-n-prop-1'-oxy)-6-isopropylidenhydrazinopyridazine der allgemeinen Formel I sind die Dihydrochloride.

Besonders bevorzugte Reste, für welche R stehen kann, sind tert.-Butyl-, n-But-1-yl-, n-But-2-yl-, Isopropyl-, Benzyl-, 2-Phenyläth-1-yl-, 3-Phenyl-n-prop-2-yl-, 4-Phenyl-n-but-2-yl- und 2-[3',4'-(Dimethoxy)-phenyl]-äth-1-ylreste.

Besonders bevorzugte erfindungsgemässe Verbindungen sind

3-(3'-tert.-Butylamino-2'-hydroxy-n-prop-1'-oxy)-6-isopropylidenhydrazinopyridazin,

3-(3'-Isopropylamino-2'-hydroxy-n-prop-1'-oxy)-6-isopropylidenhydrazinopyridazin,

3-[3'-(n-But-2''-ylamino)-2'-hydroxy-n-prop-1'-oxy]-6-isopropylidenhydrazinopyridazin,

3-/3'-[2''-(3''',4'''-/Dimethoxy/-phenyl)-äth-1''-ylamino]-2'-hydroxy-n-prop-1'oxy/-6-isopropylidenhydrazinopyridazin und

3-[3'-(4''-Phenyl-n-but-2''-ylamino)-2'-hydroxy-n-prop-1'-oxy]-6-isopropylidenhydrazinopyridazin

sowie ihre Salze, insbesondere die Hydrochloride.

Gegenstand der Erfindung ist auch ein Verfahren zur Herstellung der erfindungsgemässen Verbindungen, welches dadurch gekennzeichnet ist, dass in an sich bekannter Weise 3,6-Dichlorpyridazin der Formel II

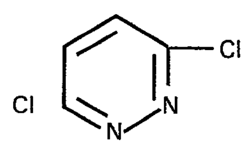

(II)

mit Isopropylidenglycerin der Formel III

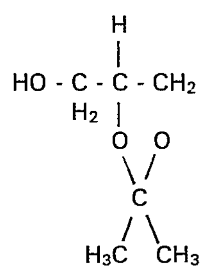

(III)

in Gegenwart einer starken Base bei Temperaturen von 30 bis 80°C in einem aprotonischen apolaren Lösungsmittel zu 3-Chlor-6-(2',2'-dimethyl-1',3'-dioxolan-4'-ylmethoxy)-pyridazin der Formel IV

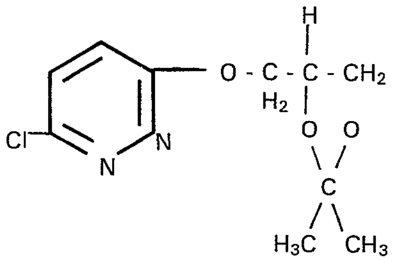

(IV)

umgesetzt wird, das letztere mit Hydrazin bei Temperaturen von 60 bis 90°C und anschliessend mit Aceton bei Raumtemperatur bis 60°C, jeweils in protonischen Lösungsmitteln behandelt und die Reaktionsmischung bei erhöhter Temperatur angesäuert wird, die erhaltenen Diole 3-(2',3'-Dihydroxy-n-prop-1'-oxy)-6-isopropylidenhydrazinopyridazine der allgemeinen Formel V

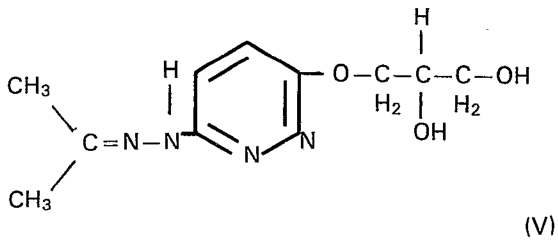

(V)

mit Orthocarbonsäurealkylestern der allgemeinen Formel VI

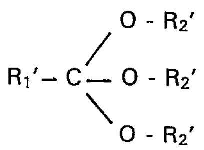

(VI)

worin $R_1'$ für Wasserstoff, einen Alkylrest mit 1 bis 3 Kohlenstoffatomen oder einen Phenylrest steht und $R_2'$ einen Alkylrest mit 1 bis 3 Kohlenstoffatomen oder einen Phenylrest bedeutet, in aprotonischen Lösungsmitteln bei Temperaturen von 60 bis 110°C zu, gegebenenfalls am Dioxolanring 2'-alkylsubstituierten, 3-(2'-Alkoxydioxolan-4'-ylmethoxy)-6-isopropylidenhydrazinopyridazinen der allgemeinen Formel VII

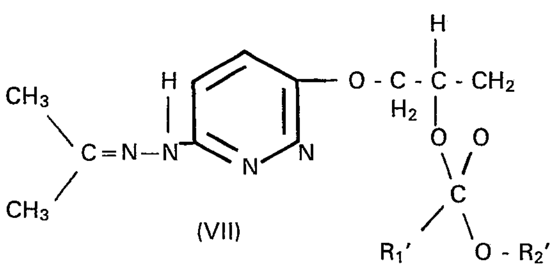

(VII)

worin $R_1'$ und $R_2'$ wie oben festgelegt sind, umgesetzt werden, die letzteren mit Trialkylsilylhalogeniden der allgemeinen Formel VIII

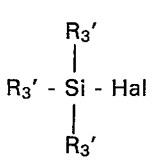

(VIII)

worin $R_3'$ für einen Alkylrest mit 1 bis 4, insbesondere 1 oder 2, Kohlenstoffatomen steht und Hal Chlor, Brom oder Jod bedeutet, bei Temperaturen von 20 bis 50°C in Chlor enthaltenden aprotonischen Lösungsmitteln zu 3-(2'-Acyloxy-3'-halogen-n-prop-1'-oxy)-6-isopropylidenhydrazinopyridazinen der allgemeinen Formel IX

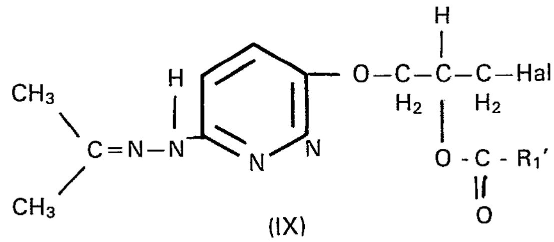

(IX)

worin $R_1'$ und Hal wie oben festgelegt sind, umgesetzt werden und in an sich bekannter Weise die letzteren mit Aminen der allgemeinen Formel X

$$H_2N - R \qquad (X)$$

worin R wie oben festgelegt ist, in protonischen Lösungsmitteln in Gegenwart von Alkalihydroxyden umgesetzt werden, worauf in an sich bekannter Weise gegebenenfalls die erhaltenen 3-Alkoxy-6-isopropylidenhydrazinopyridazine der allgemeinen Formel I mit Säuren in Säureadditionssalze überführt werden bzw. gegebenenfalls die erhaltenen Säureadditionssalze der 3-Alkoxy-6-isopropylidenhydrazinopyridazine der allgemeinen Formel I in die freien 3-Alkoxy-6-isopropylidenhydrazinopyridazine der allgemeinen Formel I oder in andere Säureadditionssalze überführt werden und/oder gegebenenfalls die erhaltenen Mischungen der optisch aktiven Isomere der 3-Alkoxy-6-isopropylidenhydrazinopyridazine der allgemeinen Formel I in die optisch aktiven Isomere gespalten werden bzw. die erhaltenen optisch aktiven Isomere der 3-Alkoxy-6-isopropylidenhydrazinopyridazine der allgemeinen Formel I racemisiert werden.

Vorteilhaft wird als starke Base zur Umsetzung des 3,6-Dichlorpyridazins der Formel II mit dem Isopropylidenglycerin der Formel III Natriumtert.-butylat oder Natriumhydrid verwendet. Als aprotonische(s) apolare(s) [nicht-polare(s)] Lösungsmittel kann bzw. können bei dieser Umsetzung vorteilhaft Toluol, Benzol und/oder 1,2-Dimethoxyäthan verwendet werden.

Vorteilhaft kann bzw. können bei der Umsetzung der 3-Chlor-6-(2',2'-dimethyl-1',3'-dioxolan-4'-ylmethoxy)-pyridazine mit dem Hydrazin und Aceton als protonisches Lösungsmittel 1 oder mehr Alkohole mit niederem Molekulargewicht, wie Äthyl- und/oder n-Propylalkohol, verwendet werden. Die erhaltenen 3-Isopropylidenhydrazino-6-(2',2'-dimethyl-1',3'-dioxolan-4'-ylmethoxy)-pyridazine der allgemeinen Formel XI (siehe Reaktionsschema Seite 5) werden vorzugsweise nicht abgetrennt, sondern die Reaktionsmischung wird unmittelbar in der Wärme durch Zugabe von starken Säuren, beispielsweise Salzsäure oder p-Toluolsulfonsäure, angesäuert. Vorzugsweise werden dabei Reaktionstemperaturen von 40 bis 60°C angewandt.

Vorteilhaft kann bzw. können bei der Umsetzung der 3-(2',3'-Dihydroxy-n-prop-1'-oxy)-6-isopropyl-

idenhydrazinopyridazine der allgemeinen Formel V mit den Orthocarbonsäureestern der allgemeinen Formel VI als aprotonische(s) Lösungsmittel Toluol, Benzol und/oder Xylol verwendet werden. Bei dieser Umsetzung scheiden sich die, gegebenenfalls 2'-alkylsubstituierten, 3-(2'-Alkoxydioxolan-4'-ylmethoxy)-6-isopropylidenhydrazinopyridazine der allgemeinen Formel VII ab.

Bei der Umsetzung der, gegebenenfalls am Dioxolanring 2'-alkylsubstituierten, 3-(2'-Alkoxydioxolan-4'-ylmethoxy)-6-isopropylidenhydrazinopyridazine der allgemeinen Formel VII mit den Trialkylsilylhalogeniden der allgemeinen Formel VIII kann bzw. können vorteilhaft als Chlor enthaltende(s) aprotonische(s) Lösungsmittel Methylenchlorid und/oder Chloroform verwendet werden.

Vorteilhaft kann bzw. können bei der Umsetzung der 3-(2'-Acyloxy-3'-halogen-n-prop-1'-oxy)-6-isopropylidenhydrazinopyridazine der allgemeine Formel IX mit den Aminen der allgemeinen Formel X als protonische(s) Lösungsmittel 1 oder mehr niedermolekulare Alkohole verwendet werden.

Die Umsetzungen des erfindungsgemässen Verfahrens können durch das folgende Reaktionsschema dargestellt werden.

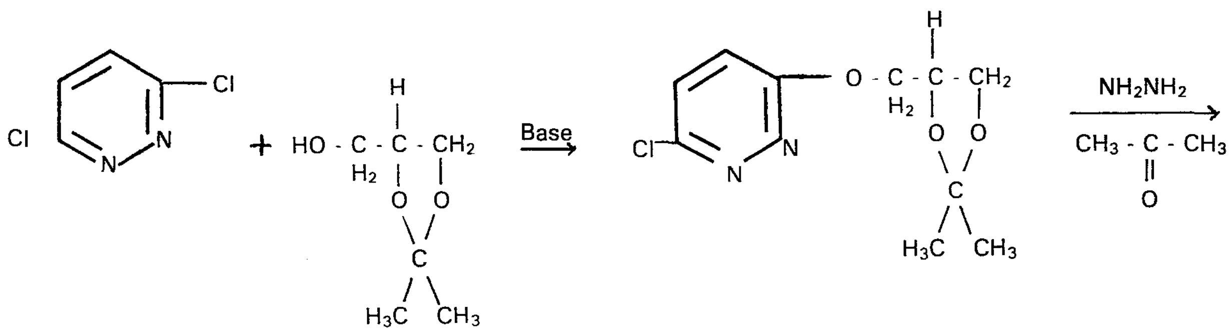


(II) (III) (IV)

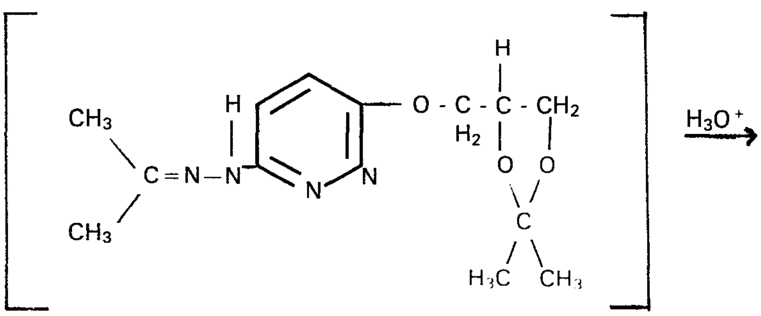


(IX)

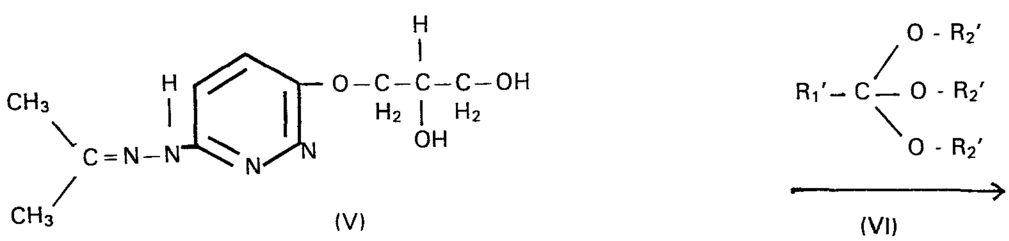

(V) (VI)

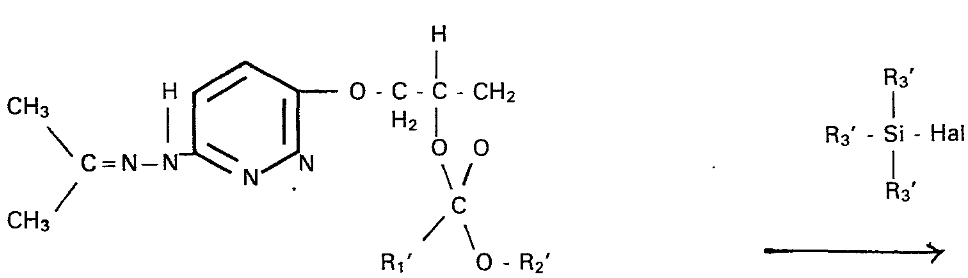

(VII) (VIII)

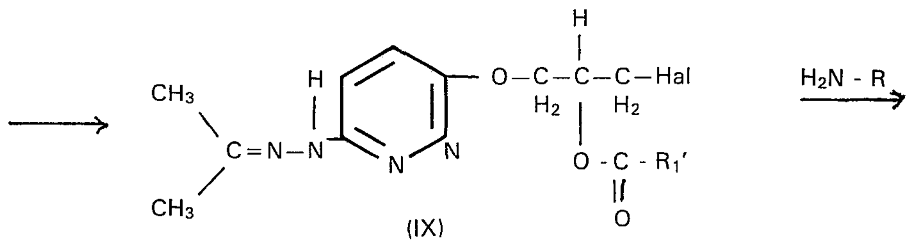


(IX)

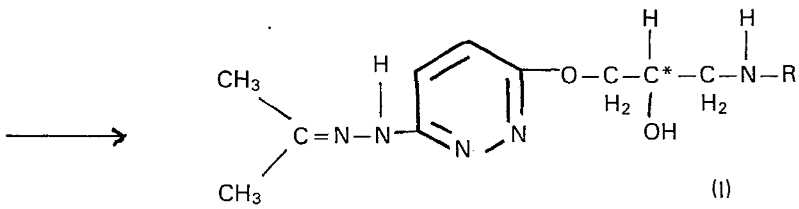

(I)

Ferner sind erfindungsgemäss Arzneimittel, welche 1 oder mehr der erfindungsgemässen Verbindungen als Wirkstoff bzw. Wirkstoffe, gegebenenfalls zusammen mit 1 oder mehr üblichen pharmazeutischen Konfektionierungsmitteln, enthalten, vorgesehen. Beispiele für solche sind Verdünnungsmittel.

Die erfindungsgemässen Verbindungen haben nämlich wie bereits erwähnt wertvolle pharmakologische, insbesondere blutdrucksenkende und gefässerweiternde Wirkungen, wobei sie bei den wirksamen Dosen praktisch frei von Tachykardiewirkungen sind. Ihre blutdrucksenkende Wirksamkeit ist auf eine gefässkrampflösende bzw. gefässspasmolytische Wirkung zurückzuführen und diese Wirksamkeit ist infolge des Vorliegens einer bestimmten $\beta$-blockierenden Wirksamkeit nicht von einer Erhöhung der Herzfrequenz bzw. Pulszahl begleitet, wie sie bei fast allen gefässerweiternden Mitteln auftritt.

Die Wirkung der erfindungsgemässen Verbindungen und von anerkannten gut wirksamen Vergleichssubstanzen gleicher Wirkung, nämlich des blutdrucksenkenden und gefässerweiternden Mittels 1-Hydrazinophthalazin (Hydralazin), des blutdrucksenkenden $\beta$-blockierenden Mittels 1-Isopropylamino-3-(1′-naphthyloxy)-n-propan-2-ol (Propanolol) und des blutdrucksenkenden $\alpha$, $\beta$-blockierenden Mittels 5-/1-Hydroxy-2-[(1-methyl-3-phenyl-n-propyl)-amino]-äthyl/-salicylamid (Labetalol) wurde insbesondere in den folgenden Hinsichten untersucht.

Die Wirkung auf den arteriellen Blutdruck und die Herzfrequenz wurde bei peroraler Verabreichung an SHR-Ratten mit genetisch bedingtem hohem Blutdruck nach der in Pharm. Research Comm. 8, 295 (1976) beschriebenen Verfahrensweise untersucht. In der folgenden Tabelle sind die durch graphische Auswertung erhaltenen $ED_{25}$-Werte im Zeitpunkt der höchsten Wirkung, welche die Menge der Verbindung, die einen Blutdruckabfall von 25% bzw. eine Erhöhung der Herzfrequenz um 25% herbeizuführen vermag, wiedergeben, zusammengestellt.

Die gefässkrampflösende bzw. gefässspasmolytische Wirksamkeit wurde durch Ermittlung der unmittelbaren entspannenden Wirkung auf den glatten Gefässmuskel in vitro an Streifen von Kaninchenhauptschlagadern, bei welchen der Krampf mit Kaliumchlorid nach der in The Journ. of Pharm. and Exper. Therapeutics 205, 2, 441 (1978) beschriebenen Verfahrensweise herbeigeführt wurde, bestimmt. Die Verbindungen wurden in aufeinanderfolgend kumulierten Molkonzentrationen verabreicht und ihre Wirksamkeit wurde nach graphischer Auswertung als wirksame Molkonzentration ($EC_{50}$-Wert) ausgedrückt. Der $EC_{50}$-Wert stellt die Konzentration der jeweiligen Verbindung, welche 50% der Streifen von Kaninchenhauptschlagadern, bei welchen mit einer 30millimolaren Kaliumchloridlösung Krämpfe herbeigeführt wurden, zu entspannen vermag, dar. Auch die Ergebnisse dieser Versuche sind in der folgenden Tabelle zusammengestellt.

Die $\beta$-blockierende Wirksamkeit wurde in vitro an aus Meerschweinchen isolierten Herzvorkammern bzw. Atrien, welche unter Verwendung von DL-1-(3′,4′-Dihydroxyphenyl)-2-isopropylaminoäthanolhydrochlorid (Isoprenalinhydrochlorid) als Agonist in aufeinanderfolgend kumulierten Molkonzentrationen erregt wurden, bestimmt. Die zu untersuchenden Verbindungen wurden in einer einzigen Dosis bzw. Konzentration von $10^{-4}$ Mol verabreicht und ihre $\beta$-blockierende Wirksamkeit wurde durch Ermittlung des Molkonzentrationsverhältnisses ($MV_4$-Verhältnis), welches das Verhältnis der wirksamen Molkonzentration ($EC_{50}$-Wert) des Agonisten in Gegenwart der zu untersuchenden Verbindung in einer Dosis bzw. Konzentration von $10^{-4}$ Mol zur wirksamen Molkonzentration ($EC_{50}$-Wert) des Agonisten in Abwesenheit der zu untersuchenden Verbindung darstellt, bestimmt. Dabei stellt der durch graphische Auswertung ermittelte $EC_{50}$-Wert die Molkonzentration des Agonisten, welche eine 50%ige Aktivierung des Organs im Vergleich zur höchstmöglichen Aktivierung herbeizuführen vermag, dar. Die erhaltenen Ergebnisse sind ebenfalls in der folgenden Tabelle zusammengestellt.

Die als intraperitonealer $LD_{50}$-Wert ausgedrückte akute Toxizität wurde an Mäusen nach der Verfahrensweise von Irwin [Gordon Res. Conf. Med. Chem. New London, N. H., 3/7-8, 133 (1959)] bestimmt. Auch diese Werte sind in der folgenden Tabelle zusammengestellt.

Tabelle

| Beispiel Nr. | Verbindung Bezeichnung | Intraperitonealer $LD_{50}$-Wert an Mäusen in mg/kg | Peroraler $ED_{25}$-Wert den arteriellen Blutdruck bei SHR-Ratten betreffend in mg/kg | Peroraler $ED_{25}$-Wert die Herzfrequenz bei SHR-Ratten betreffend in mg/kg | $EC_{50}$-Wert die krampflösende Wirkung bei Kaninchenhauptschlagadern betreffend in Molkonzentrationen | $MV_4$-Verhältnis die -blockierende Wirksamkeit bei Meerschweinchenherzvorkammern betreffend | Therapeutischer Index bezüglich der Senkung des arteriellen Blutdruckes $\frac{LD_{50}\text{-Wert}}{ED_{25}\text{-Wert}}$ | Therapeutischer Index bezüglich der Nichtbeeinflussung der Herzfrequenz $LD_{50}$-Wert . $ED_{25}$-Wert | Therapeutischer Index bezüglich der gefässkrampflösenden Wirkung $\frac{LD_{50}\text{-Wert}}{EC_{50}\text{-Wert}}$ | Therapeutischer Index bezüglich der -blockierenden Wirksamkeit $LD_{50}$-Wert . $MV_4$-Verhältnis |
|---|---|---|---|---|---|---|---|---|---|---|
| 1 | 3-(3'-tert.-Butylamino-2'-hydroxy-n-prop-1'-oxy)-6-isopropylidenhydrazinopyridazindihydrochlorid | 500 | 50 | > 100 | $27 \times 10^{-4}$ | 78 | 10 | > 50000 | 185185 | 39000 |
| 2 | 3-(3'-Isopropylamino-2'-hydroxy-n-prop-1'-oxy)-6-isopropylidenhydrazinopyridazindihydrochlorid | 500 | 350 | > 100 | $17 \times 10^{-4}$ | 77 | 1,4 | > 50000 | 294118 | 37500 |
| 8 | 3-[3'-(n-But-2''-ylamino)-2'-hydroxy-n-prop-1'-oxy]-6-isopropylidenhydrazinopyridazindihydrochlorid | 300 | 25 | > 50 | – | 72 | 12 | > 15000 | – | 21600 |
| 7 | 3-/3'-[2''-(3''',4'''-/Dimethoxy/-phenyl)-äth-1''-ylamino]-2'-hydroxy-n-prop-1'-oxy/-6-isopropylidenhydrazinopyridazindihydrochlorid | 300 | 39 | > 60 | – | 531 | 7,7 | > 18000 | – | 159300 |
| 6 | 3-[3'-(4''-Phenyl-n-but-2''-ylamino)-2'-hydroxy-n-prop-1'-oxy]-6-iso-propylidenhydrazinopyridazindihydrochlorid | 200 | 17 | > 40 | – | 80 | 11,8 | > 8000 | – | 16000 |

Tabelle (Fortsetzung)

| Verbindung Beispiel Nr. | Bezeichnung | Intra-peritonealer $LD_{50}$-Wert an Mäusen in mg/kg | Peroraler $ED_{25}$-Wert den arteriellen Blutdruck bei SHR-Ratten betreffend in mg/kg | Peroraler $ED_{25}$-Wert die Herzfrequenz bei SHR-Ratten betreffend in mg/kg | $EC_{50}$-Wert die krampflösende Wirkung bei Kaninchenhauptschlagadern betreffend in Molkonzentrationen | $MV_4$-Verhältnis die -blockierende Wirksamkeit bei Meerschweinchenherzvorkammern betreffend | Therapeutischer Index bezüglich der Senkung des arteriellen Blutdruckes $\frac{LD_{50}\text{-Wert}}{ED_{25}\text{-Wert}}$ | Therapeutischer Index bezüglich der Nichtbeeinflussung der Herzfrequenz $LD_{50}$-Wert . $ED_{25}$-Wert | Therapeutischer Index bezüglich der gefäss krampflösenden Wirkung $\frac{LD_{50}\text{-Wert}}{EC_{50}\text{-Wert}}$ | Therapeutischer Index bezüglich der -blockierenden Wirksamkeit $LD_{50}$-Wert . $MV_4$-Verhältnis |
|---|---|---|---|---|---|---|---|---|---|---|
| Vergleichssubstanz A | 1-Hydrazinophthalazin [Hydralazin] | 100 | 8,1 | 6,0 | $6{,}8 \times 10^{-4}$ | 1 | 12,3 | 600 | 147058 | 100 |
| Vergleichssubstanz B | 1-Isopropylamino-3--(1'-naphthyloxy)-n--propan-2-ol [Propranolol] | 90 | > 400 | > 100 | $>1 \times 10^{-2}$ | 6 500 | < 0,2 | > 9000 | 9000 | 585000 |
| Vergleichssubstanz C | 5-/1-Hydroxy-2-[(1--methyl-3-phenyl-n-propyl)--amino]-äthyl/-salicylamid [Labetalol] | 300 | 400 | > 100 | $> 1 \times 10^{-2}$ | 12590 | 0,75 | > 30000 | 30000 | 3777000 |

> = keine Erhöhung der Herzfrequenz bei der untersuchten Höchstdosis

Aus der obigen Tabelle geht die Überlegenheit der erfindungsgemässen Verbindungen als blutdrucksenkende und gefässkrampflösende Mittel hervor. So haben die erfindungsgemässen Verbindungen um 1 bis 2 Grössenordnungen höhere therapeutische Indices bezüglich der Senkung des arteriellen Blutdruckes als die Vergleichssubstanzen 1-Isopropylamino-3-(1'-naphthyloxy)-n-propan-2-ol (Propanolol) und 5-/1-Hydroxy-2-[(1-methyl-3-phenyl-n-propyl)-amino]-äthyl/-salicylamid (Labetalol) und mit einer einzigen Ausnahme [3-(3'-Isopropylamino-2'-hydroxy-n-prop-1'-oxy)-6-isopropylidenhydrazinopyridazindihydrochlorid] nahezu gleiche therapeutische Indices bezüglich der Senkung des arteriellen Blutdruckes wie die Vergleichssubstanz 1-Hydrazinophthalazin (Hydralazin). Aber auch diese letztgenannte erfindungsgemässe Verbindung bringt gegenüber dem 1-Hydrazinophthalazin (Hydralazin) den entscheidenden grossen Vorteil mit sich, dass ihr therapeutischer Index bezüglich der Nichtbeeinflussung der Herzfrequenz um 2 Grössenordnungen höher als der des 1-Hydrazinophthalazines (Hydralazines) ist. Dabei sind die diesbezüglichen therapeutischen Indices aller erfindungsgemässen Verbindungen um 1 bis 2 Grössenordnungen höher als der diesbezügliche therapeutische Index des 1-Hydrazinophthalazines (Hydralazines). Hinzu kommt noch, dass die therapeutischen Indices der erfindungsgemässen Verbindungen auch bezüglich der gefässkrampflösenden Wirkung und bezüglich der $\beta$-blockierenden Wirksamkeit wesentlich höher als die des 1-Hydrazinophthalazines (Hydralazines) sind. In der letztgenannten Hinsicht sind die therapeutischen Indices der ersteren sogar um 2 bis 3 Grössenordnungen höher als der therapeutische Index des letzteren. Die therapeutischen Indices bezüglich der gefässkrampflösenden Wirkung der erfindungsgemässen Verbindungen sind auch um Grössenordnungen höher als die der Vergleichssubstanzen 1-Isopropylamino-3-(1'-naphthyloxy)-n-propan-2-ol (Propanolol) und 5-/1-Hydroxy-2-[(1-methyl-3-phenyl-n-propyl)-amino]-äthyl/-salicylamid (Labetalol), welche wie bereits erwähnt ohnehin hinsichtlich der Senkung des arteriellen Blutdruckes den ersteren bei weitem unterlegen sind.

Gegenüber den aus der DE-A-2 406 930 bekannten substituierten 3-Amino-2-hydroxy-n-prop-1-oxyheterocyclen, von denen sich die erfindungsgemässen 3-(3'-Amino-2'-hydroxy-n-prop-1'-oxy)-6-isopropylidenhydrazinopyridazine massgeblich durch das Vorliegen der Isopropylidenhydrazinogruppe in der 6-Stellung des Pyridazinringes unterscheiden, mit ihren $\beta$-rezeptorblockierenden und stimulierenden Wirkungen ohne gefässerweiternde Wirkung haben die erfindungsgemässen Verbindungen eine gefässerweiternde Wirkung mit nur einer $\beta$-blockierenden Wirkungskomponente, also eine grundlegend andere Wirkung.

Von den in der BE-A-830 158 und DE-A-2 556 918 beschriebenen 3-Phenyl-6-hydrazinopyridazinen unterscheiden sich die erfindungsgemässen 3-(3'-Amino-2'-hydroxy-n-prop-1'-oxy)-6-isopropylidenhydrazinopyridazine massgeblich durch das Fehlen des Phenylrestes am Pyridazinring und das Vorliegen eines 3-Amino-2-hydroxy-n-prop-1-oxyrestes an dessen Stelle sowie die festgelegte Substitution der Hydrazinogruppe.

Gegenüber den aus der DE-A-2 154 245 bekannten Pyridazinderivaten, von denen sich die erfindungsgemässen 3-(3'-Amino-2'-hydroxy-n-prop-1'-oxy)-6-isopropylidenhydrazinopyridazine massgeblich durch das Vorliegen einer 3-Amino-2-hydroxy-n-prop-1-oxygruppe am Pyridazinring statt der substituierten Aminogruppe unterscheiden, haben die erfindungsgemässen 3-(3'-Amino-2'-hydroxy-n-prop-1'-oxy)-6-isopropylidenhydrazinopyridazine den grossen Vorteil der $\beta$-blockierenden Wirkung bei Fehlen der Nebenwirkungen von der Art der Tachykardie.

Die Erfindung wird anhand der folgenden Beispiele näher erläutert.

*Beispiel 1*

3-(3'-tert.-Butylamino-2'-hydroxy-n-prop-1'-oxy)-6-isopropylidenhydrazinopyridazindihydrochlorid

Es wurde eine Lösung von 31,1 $cm^3$ Isopropylidenglycerin (2,2-Dimethyl-4-hydroxymethyl-1,3-dioxolan) in 80 $cm^3$ Dimethoxyäthan zu einer Suspension von 13,5 g 50%igem Natriumhydrid in 120 $cm^3$ 1,2-Dimethoxyäthan während 1 Stunde unter Rühren bzw. Schütteln zugegeben. Die erhaltene Mischung wurde noch 3 Stunden bei Raumtemperatur gerührt bzw. geschüttelt, worauf eine Lösung von 37,2 g 3,6-Dichlorpyridazin in 100 $cm^3$ 1,2-Dimethoxyäthan während 1 Stunde zugesetzt wurde. Die Reaktionsmischung wurde 2 Stunden lang auf 60°C erwärmt und über Nacht bei Raumtemperatur gerührt bzw. geschüttelt, worauf das Lösungsmittel unter Vakuum abgedampft und der Rückstand in 400 $cm^3$ Wasser aufgenommen und mit 1000 $cm^3$ Äthyläther extrahiert wurde. Die ätherische Lösung wurde über wasserfreiem Natriumsulfat wasserfrei gemacht und unter Vakuum zur Trockne eingedampft und der erhaltene Rückstand wurde aus n-Hexan kristallisiert. So wurden 55 g (90% der Theorie) 3-Chlor-6-(2',2'-dimethyl-1',3'-dioxolan-4'-ylmethoxy)-pyridazin mit einem Schmelzpunkt von 68°C erhalten.

40 g des so erhaltenen 3-Chlor-6-(2',2'-dimethyl-1',3'-dioxolan-4'-ylmethoxy)-pyridazines, 200 $cm^3$ Äthylalkohol und 300 $cm^3$ Hydrazinhydrat wurden 5 Stunden lang unter Rückfluss zum Sieden erhitzt. Die Lösung wurde unter Vakuum zur Trockne eingedampft und der Rückstand wurde in eine Mischung von 50 $cm^3$ Methylalkohol und 500 $cm^3$ Aceton aufgenommen und über Nacht bei Raumtemperatur gerührt bzw. geschüttelt. Der durch Eindampfen zur Trockne unter Vakuum erhaltene Rückstand wurde in 100 $cm^3$ Wasser aufgenommen und der pH-Wert wurde mit einer 20%igen Salzsäure auf 1 eingestellt. Das erhaltene Produkt wurde 1 Stunde lang auf 60°C erwärmt, dann auf Raumtemperatur gekühlt und durch Zugabe von Natrumbicarbonat neutralisiert. Dann wurde die Mischung 6mal mit je 50 $cm^3$ Diäthyläther extrahiert und die wässrige Schicht wurde erneut mit Salzsäure bis zur Erreichung des pH-Wertes von 1 angesäuert und zur Trockne eingedampft. Der erhaltene Rückstand wurde aus 500 $cm^3$ einer Mischung aus Äthylalkohol und Aceton

im Volumenverhältnis von 90 : 10 kristallisiert. So wurden 16 g (36% der Theorie) 3-(2',3'-Dihydroxy--n-prop-1'-oxy)-6-isopropylidenhydrazinopyridazin-hydrochlorid mit einem Schmelzpunkt von 187 bis 189°C erhalten. Es wurde eine Mischung aus 16,5 g wie vorstehend beschrieben erhaltenem 3-(2',3'--Dihydroxy-n-prop-1'-oxy)-6-isopropylidenhydrazinopyridazinhydrochlorid, 100 $cm^3$ Toluol, 9 $cm^3$ Triäthylamin und 11,3 $cm^3$ Orthoessigsäuremethylester 3 Stunden lang auf 100°C erhitzt. Nach dem Kühlen auf Raumtemperatur wurde die Reaktionsmischung mit 100 $cm^3$ Diäthyläther verdünnt und 2mal mit je 30 $cm^3$ Wasser gewaschen, worauf die organische Schicht über wasserfreiem Natriumsulfat wasserfrei gemacht und zur Trockne eingedampft wurde. Der Rückstand wurde aus einer Mischung aus Benzol und n-Hexan kristallisiert. So wurden 11 g (61,9% der Theorie) 3-(2'-Methyl-2'-methoxydioxolan-4'-ylmethoxy)-6-isopropylidenhydrazinopyridazin mit einem Schmelzpunkt von 119 bis 121°C erhalten.

Es wurde eine Mischung aus 8,9 g des so erhaltenen 3-(2'-Methyl-2'-methoxydioxolan-4'-ylmethoxy)-6-isopropylidenhydrazinopyridazines, 50 $cm^3$ Methylenchlorid und 5,9 $cm^3$ Trimethylsilylchlorid 2 Stunden lang auf 40°C erwärmt. Dann wurde gekühlt und mit einer gesättigten Natriumbicarbonatlösung gewaschen, über wasserfreiem Natriumsulfat wasserfrei gemacht und zur Trockne eingedampft. Der ölige Rückstand wurde an einer Silicagel®säule gereinigt. So wurden 2,2 g (25% der Theorie) 3-(2'-Acetoxy-3'-chlor-n-prop-1'-oxy)-6-isopropylidenhydrazinopyridazin mit einem Schmelzpunkt von 94 bis 96°C (nach dem Umkristallisieren aus n-Hexan) erhalten.

Es wurde eine Lösung von 0,4 g Natriumhydroxyd in 20 $cm^3$ Methylalkohol zu einer Mischung aus 2,4 g wie vorstehend beschrieben erhaltenem 3-(2'-Acetoxy-3'-chlor-n-prop-1'-oxy)-6-isopropylidenhydrazinopyridazin, 30 $cm^3$ Methylalkohol und 20 $cm^3$ tert.-Butylamin zugegeben. Die Mischung wurde 24 Stunden lang bei Raumtemperatur gerührt bzw. geschüttelt und zur Trockne eingedampft und der Rückstand wurde in 50 $cm^3$ Chloroform aufgenommen und 3mal mit je 50 $cm^3$ Wasser gewaschen und die organische Schicht über wasserfreiem Natriumsulfat wasserfrei gemacht und unter Vakuum eingedampft. Der erhaltene Rückstand wurde in 20 $cm^3$ Äthyläther aufgenommen und filtriert. Das feste Material wurde in 10 $cm^3$ Chlorwasserstoffgas enthaltendem Methylalkohol gelöst und der pH-Wert wurde auf 1 eingestellt. Die Mischung wurde zur Trockne eingedampft und der Rückstand wurde aus Isopropylalkohol kristallisiert. So wurden 1,76 g (60% der Theorie) 3-(3'-tert.-Butylamino-2'-hydroxy-n-prop-1'-oxy)-6-isopropylidenhydrazinopyridazindihydrochlorid mit einem Schmelzpunkt von 238 bis 241°C (unter Zersetzung) erhalten.

*Beispiel 2*

3-(3'-Isopropylamino-2'-hydroxy-n-prop-1'-oxy)--6-isopropylidenhydrazinopyridazindihydrochlorid

Es wurde wie im Beispiel 1 beschrieben erhaltenes 3-(2'-Acetoxy-3'-chlor-n-prop-1'-oxy)-6-isopropylidenhydrazinopyridazin analog der im Beispiel 1 beschriebenen Verfahrensweise mit Isopropylamin umgesetzt. So wurde 3-(3'-Isopropylamino-2'-hydroxy-n-prop-1'-oxy)-6-isopropylidenhydrazinopyridazindihydrochlorid mit einem Schmelzpunkt von 218 bis 220°C (unter Zersetzung) (nach dem Kristallisieren aus einem Gemisch aus Äthylalkohol und Aceton) in einer Ausbeute von 40% der Theorie erhalten.

Beim Arbeiten in analoger Weise wie im Beispiel 1 angegeben wurden aus wie im Beispiel 1 beschrieben erhaltenem 3-(2'-Acetoxy-3'-chlor-n-prop-1'--oxy)-6-isopropylidenhydrazinopyridazin durch Umsetzen mit den entsprechenden Aminen die in den folgenden Beispielen 3 bis 8 angegebenen Verbindungen erhalten.

*Beispiel 3*

3-(3'-Benzylamino-2'-hydroxy-n-prop-1'-oxy)-6--isopropylidenhydrazinopyridazindihydrochlorid

Schmelzpunkt: 216°C (unter Zersetzung).

*Beispiel 4*

3-[3'-(2''-Phenyläth-1''-ylamino)-2'-hydroxy-n--prop-1'-oxy]-6-isopropylidenhydrazinopyridazindihydrochlorid

Schmelzpunkt: 217 bis 222°C (unter Zersetzung).

*Beispiel 5*

3-[3'-(3''-Phenyl-n-prop-2''-ylamino)-2'-hydroxy--n-prop-1'-oxy]-6-isopropylidenhydrazinopyridazindihydrochlorid

Schmelzpunkt: 204 bis 207°C (unter Zersetzung).

*Beispiel 6*

3-[3'-(4''-Phenyl-n-but-2''-ylamino)-2'-hydroxy--n-prop-1'-oxy]-6-isopropylidenhydrazinopyridazindihydrochlorid

Schmelzpunkt: 170 bis 175°C (unter Zersetzung).

*Beispiel 7*

3-/3'-[2''-(3''',4'''-/Dimethoxy/-phenyl)-äth-1''--ylamino]-2'-hydroxy-n-prop-1'-oxy/-6-isopropylidenhydrazinopyridazindihydrochlorid

Schmelzpunkt: 210 bis 215°C (unter Zersetzung).

*Beispiel 8*

3-[3'-(n-But-2''-ylamino)-2'-hydroxy-n-prop-1'--oxy]-6-isopropylidenhydrazinopyridazindihydrochlorid

Schmelzpunkt: 205 bis 209°C (unter Zersetzung).

**Patentansprüche für die Vertragsstaaten:**
BE, CH, DE, FR, GB, IT, LI, NL

1. 3-(3'-Amino-2'-hydroxy-n-prop-1'-oxy)-6--isopropylidenhydrazinopyridazine der allgemeinen Formel I

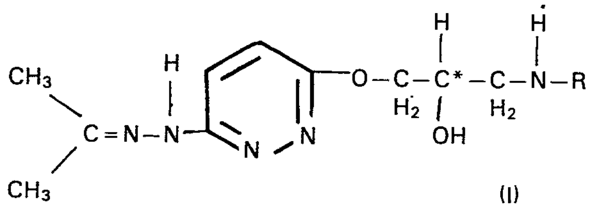

(I)

worin

R für einen Alkylrest mit 1 bis 4 Kohlenstoffatomen steht, der gegebenenfalls durch einen Phenylrest substituiert ist, welcher noch 1 bis 3 Methoxyreste tragen kann, und

das Sternchen (*) ein Asymmetriezentrum des Moleküls bedeutet,

in Form der getrennten optisch aktiven Isomeren oder von Mischungen derselben sowie ihre Salze mit pharmazeutisch brauchbaren Säuren.

2. 3-(3'-tert.-Butylamino-2'-hydroxy-n-prop-1'-oxy)-6-isopropylidenhydrazinopyridazin sowie seine Salze mit pharmazeutisch brauchbaren Säuren.

3. 3-(3'-Isopropylamino-2'-hydroxy-n-prop-1'-oxy)-6-isopropylidenhydrazinopyridazin sowie seine Salze mit pharmazeutisch brauchbaren Säuren.

4. 3-[3'-(n-But-2''-ylamino)-2'-hydroxy-n-prop-1'-oxy]-6-isopropylidenhydrazinopyridazin sowie seine Salze mit pharmazeutisch brauchbaren Säuren.

5. 3-/3'-[2''-(3''',4'''-/Dimethoxy/-phenyl)-äth-1''-ylamino]-2'-hydroxy-n-prop-1'-oxy/-6-isopropylidenhydrazinopyridazin sowie seine Salze mit pharmazeutisch brauchbaren Säuren.

6. 3-[3'-(4''-Phenyl-n-but-2''-ylamino)-2'-hydroxy-n-prop-1'-oxy]-6-isopropylidenhydrazinopyridazin sowie seine Salze mit pharmazeutisch brauchbaren Säuren.

7. Salze der 3-(3'-Amino-2'-hydroxy-n-prop-1'-oxy)-6-isopropylidenhydrazinopyridazine nach Anspruch 1 bis 6, dadurch gekennzeichnet, dass sie Dihydrochloride sind.

8. Verfahren zur Herstellung der Verbindungen nach Anspruch 1 bis 7, dadurch gekennzeichnet, dass man in an sich bekannter Weise 3,6-Dichlorpyridazin der Formel II

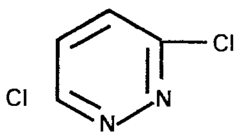

(II)

mit Isopropylidenglycerin der Formel III

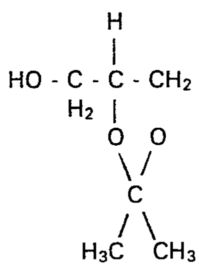

(III)

in Gegenwart einer starken Base bei Temperaturen von 30 bis 80°C in einem aprotonischen apolaren Lösungsmittel zu 3-Chlor-6-(2',2'-dimethyl-1',3'-dioxolan-4'-ylmethoxy)-pyridazin der Formel IV

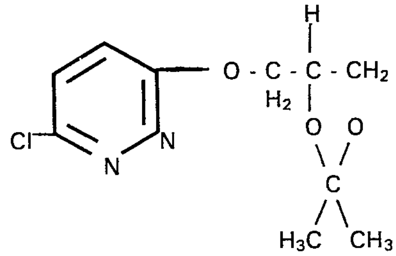

(IV)

umsetzt, das letztere mit Hydrazin bei Temperaturen von 60 bis 90°C und anschliessend mit Aceton bei Raumtemperatur bis 60°C, jeweils in protonischen Lösungsmitteln behandelt und die Reaktionsmischung bei erhöhter Temperatur ansäuert, die erhaltenen Diole 3-(2',3'-Dihydroxy-n-prop-1'-oxy)-6-isopropylidenhydrazinopyridazine der allgemeinen Formel V

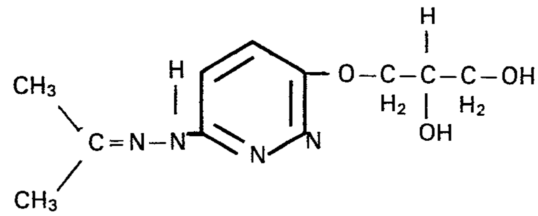

(V)

mit Orthocarbonsäurealkylestern der allgemeinen Formel VI

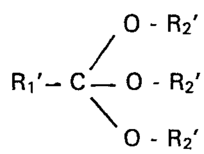

(VI)

worin $R_1'$ für Wasserstoff, einen Alkylrest mit 1 bis 3 Kohlenstoffatomen oder einen Phenylrest steht und $R_2'$ einen Alkylrest mit 1 bis 3 Kohlenstoffatomen oder einen Phenylrest bedeutet, in aprotonischen Lösungsmitteln bei Temperaturen von 60 bis 110°C zu, gegebenenfalls am Dioxolanring 2'-alkylsubstituierten, 3-(2'-Alkoxydioxolan-4'-ylmethoxy)-6-isopropylidenhydrazinopyridazinen der allgemeinen Formel VII

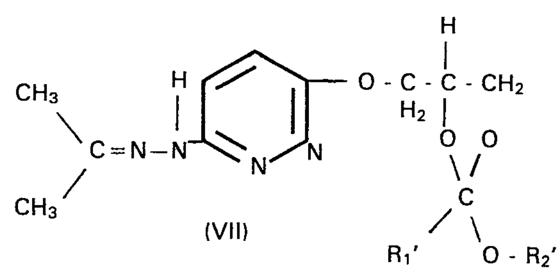

(VII)

worin $R_1'$ und $R_2'$ wie oben festgelegt sind, umsetzt, die letzteren mit Trialkylsilylhalogeniden der allgemeinen Formel VIII

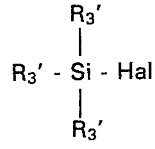

(VIII)

worin $R_3'$ für einen Alkylrest mit 1 bis 4 Kohlenstoffatomen steht und Hal Chlor, Brom oder Jod bedeutet, bei Temperaturen von 20 bis 50°C in Chlor enthaltenden aprotonischen Lösungsmitteln zu 3-(2'-Acyloxy-3'-halogen-n-prop-1'-oxy)-6-isopropylidenhydrazinopyridazinen der allgemeinen Formel IX

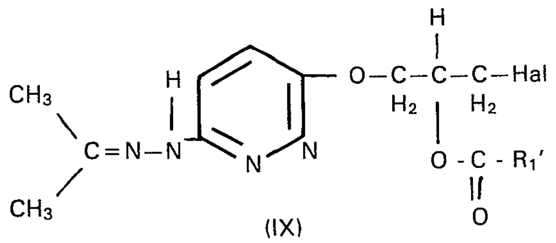

(IX)

worin $R_1'$ und Hal wie oben festgelegt sind, umsetzt und die letzteren mit Aminen der allgemeinen Formel X

$$H_2N - R \quad (X)$$

worin R wie im Anspruch 1 festgelegt ist, in protonischen Lösungsmitteln in Gegenwart von Alkalihydroxyden umsetzt, worauf man in an sich bekannter Weise gegebenenfalls die erhaltenen 3-Alkoxy-6-isopropylidenhydrazinopyridazine der allgemeinen Formel I mit Säuren in Säureadditionssalze überführt bzw. gegebenenfalls die erhaltenen Säureadditionssalze der 3-Alkoxy-6-isopropylidenhydrazinopyridazine der allgemeinen Formel I in die freien 3-Alkoxy-6-isopropylidenhydrazinopyridazine der allgemeinen Formel I oder in andere Säureadditionssalze überführt und/oder gegebenenfalls die erhaltenen Mischungen der optisch aktiven Isomere der 3-Alkoxy-6-isopropylidenhydrazinopyridazine der allgemeinen Formel I in die optisch aktiven Isomere spaltet bzw. die erhaltenen optisch aktiven Isomere der 3-Alkoxy-6-isopropylidenhydrazinopyridazine der allgemeinen Formel I racemisiert.

9. Arzneimittel, gekennzeichnet durch einen Gehalt an einer oder mehr Verbindungen nach Anspruch 1 bis 7 als Wirkstoff(e), gegebenenfalls zusammen mit 1 oder mehr üblichen pharmazeutischen Konfektionierungsmitteln.

**Patentansprüche für den Vertragsstaat:** AT

1. Verfahren zur Herstellung von 3-(3'-Amino-2'-hydroxy-n-prop-1'-oxy)-6-isopropylidenhydrazinopyridazinen der allgemeinen Formel I

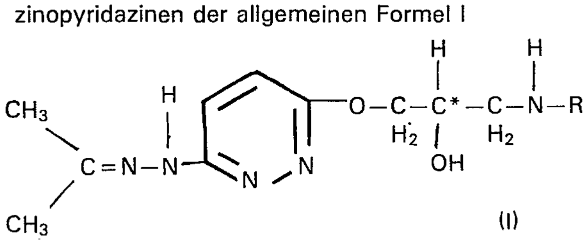

(I)

worin

R für einen Alkylrest mit 1 bis 4 Kohlenstoffatomen steht, der gegebenenfalls durch einen Phenylrest substituiert ist, welcher noch 1 bis 3 Methoxyreste tragen kann, und

das Sternchen (*) ein Asymmetriezentrum des Moleküls bedeutet,

in Form der getrennten optisch aktiven Isomeren oder von Mischungen derselben sowie ihren Salzen mit pharmazeutisch brauchbaren Säuren, dadurch gekennzeichnet, dass man in an sich bekannter Weise 3,6-Dichloridpyridazin der Formel II

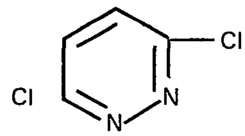

mit Isopropylidenglycerin der Formel III

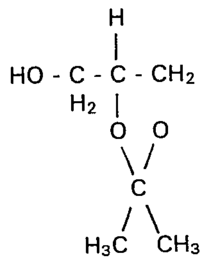

in Gegenwart einer starken Base bei Temperaturen von 30 bis 80°C in einem aprotonischen apolaren Lösungsmittel zu 3-Chlor-6-(2',2'-dimethyl-1',3'-dioxolan-4'-ylmethoxy)-pyridazin der Formel IV

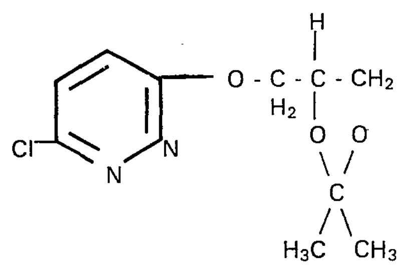

(IV)

umsetzt, das letztere mit Hydrazin bei Temperaturen von 60 bis 90°C und anschliessend mit Aceton bei Raumtemperatur bis 60°C, jeweils in protonischen Lösungsmitteln behandelt und die Reaktionsmischung bei erhöhter Temperatur ansäuert, die erhaltenen Diole 3-(2',3'-Dihydroxy-n-prop-1'-oxy)-6-isopropylidenhydrazinopyridazine der allgemeinen Formel V

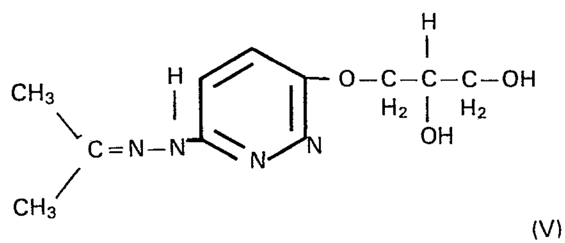

(V)

mit Orthocarbonsäurealkylestern der allgemeinen Formel VI

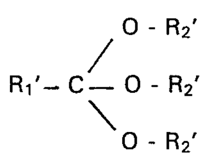

(VI)

worin $R_1'$ für Wasserstoff, einen Alkylrest mit 1 bis 3 Kohlenstoffatomen oder einen Phenylrest steht und $R_2'$ einen Alkylrest mit 1 bis 3 Kohlenstoffatomen oder einen Phenylrest bedeutet, in aprotonischen Lösungsmitteln bei Temperaturen von 60 bis 110°C zu, gegebenenfalls am Dioxolanring 2-alkylsubstituierten, 3-(2'-Alkoxydioxolan-4'-ylmethoxy)-6-isopropylidenhydrazinopyridazinen der allgemeinen Formel VII

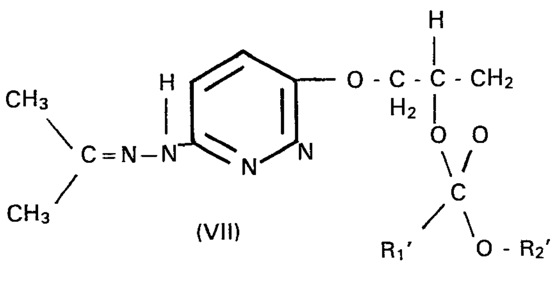

(VII)

worin $R_1'$ und $R_2'$ wie oben festgelegt sind, umsetzt, die letzteren mit Trialkylsilylhalogeniden der allgemeinen Formel VIII

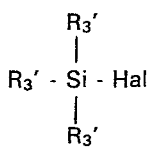

(VIII)

worin $R_3'$ für einen Alkylrest mit 1 bis 4 Kohlenstoffatomen steht und Hal Chlor, Brom oder Jod bedeutet, bei Temperaturen von 20 bis 50°C in Chlor enthaltenden aprotonischen Lösungsmitteln zu 3-(2'-Acyloxy-3'-halogen-n-prop-1'-oxy)-6-isopropylidenhydrazinopyridazinen der allgemeinen Formel IX

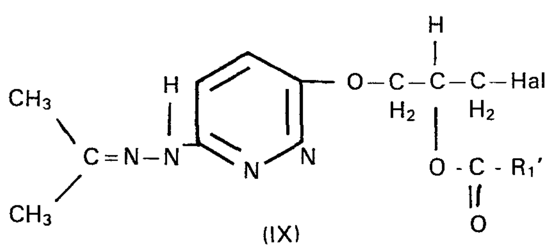

(IX)

worin $R_1'$ und Hal wie oben festgelegt sind, umsetzt und die letzteren mit Aminen der allgemeinen Formel X

$$H_2N - R \qquad X$$

worin R wie im Anspruch 1 festgelegt ist, in protonischen Lösungsmitteln in Gegenwart von Alkalihydroxyden umsetzt, worauf man in an sich bekannter Weise gegebenenfalls die erhaltenen 3-Alkoxy-6-isopropylidenhydrazinopyridazine der allgemeinen Formel I mit Säuren in Säureadditionssalze überführt bzw. gegebenenfalls die erhaltenen Säureadditionssalze der 3-Alkoxy-6-isopropylidenhydrazinopyridazine der allgemeinen Formel I in die freien 3-Alkoxy-6-isopropylidenhydrazinopyridazine der allgemeinen Formel I oder in andere Säureadditionssalze überführt und/oder gegebenenfalls die erhaltenen Mischungen der optisch aktiven Isomere der 3-Alkoxy-6-isopropylidenhydrazinopyridazine der allgemeinen Formel I in die optisch aktiven Isomere spaltet bzw. die erhaltenen optisch aktiven Isomere der 3-Alkoxy-6-isopropylidenhydrazinopyridazine der allgemeinen Formel I racemisiert.

2. Verfahren nach Anspruch 1, dadurch gekennzeichnet, dass man als Salze der 3-(3'-Amino-2'-hydroxy-n-prop-1'-oxy)-6-isopropylidenhydrazinopyridazine der allgemeinen Formel I Dihydrochloride herstellt.

3. Verfahren nach Anspruch 1 oder 2, dadurch gekennzeichnet, dass man 3-(3'-tert.-Butylamino-2'-hydroxy-n-prop-1'-oxy)-6-isopropylidenhydrazinopyridazin, 3-(3'-Isopropylamino-2'-hydroxy-n-prop-1'-oxy)-6-isopropylidenhydrazinopyridazin, 3-[3'-(n-But-2''-ylamino)-2'-hydroxy-n-prop-1'-oxy]-6-isopropylidenhydrazinopyridazin, 3-/3'-[2''-(3''',4'''-/Dimethoxy/-phenyl)-äth-1''-ylamino]-2'-hydroxy-n-prop-1'-oxy/-6-isopropylidenhydrazinopyridazin oder 3-[3'-(4''-Phenyl-n-but-2''-ylamino)-2'-hydroxy-n-prop-1'-oxy]-6-isopropylidenhydrazinopyridazin sowie ihre Salze herstellt.

**Claims for the Contracting States:**
BE, CH, DE, FR, GB, IT, LI, NL

1. 3-(3'-amino-2'-hydroxy-n-prop-1'-oxy)-6-isopropylidenehydrazinopyridazines having the general formula I

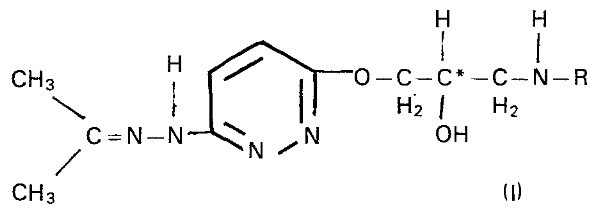

(I)

wherein
R represents an alkyl radical having from 1 to 4 carbon atoms which optionally is substituted by a phenyl radical which moreover may bear from 1 to 3 methoxy radicals and
the asterisk (*) means an asymmetry centre of the molecule
in the form of separated optically active isomers or of mixtures of them as well as their salts with pharmaceutically usable acids.

2. 3-(3'-tert.-butylamino-2'-hydroxy-n-prop-1'-oxy)-6-isopropylidenehydrazinopyridazine as well as its salts with pharmaceutically usable acids.

3. 3-(3'-isopropylamino-2'-hydroxy-n-prop-1'-oxy)-6-isopropylidenehydrazinopyridazine as well as its salts with pharmaceutically usable acids.

4. 3-[3'-(n-but-2''-ylamino)-2'-hydroxy-n-prop-1'-oxy]-6-isopropylidenehydrazinopyridazine as well as its salts with pharmaceutically usable acids.

5. 3-/3'-[2''-(3''',4'''-/dimethoxy/phenyl-eth-1''-ylamino]-2'-hydroxy-n-prop-1'-oxy/-6-isopropylidenehydrazinopyridazine as well as its salts with pharmaceutically usable acids.

6. 3-[3'-(4''-phenyl-n-but-2''-ylamino)-2'-hydroxy-n-prop-1'-oxy]-6-isopropylidenehydrazinopyridazine as well as its salts with pharmaceutically usable acids.

7. Salts of the 3-(3'-amino-2'-hydroxy-n-prop-1'-oxy)-6-isopropylidenehydrazinopyridazines according to claims 1 to 6, characterized in that they are dihydrochlorides.

8. A process for preparing the compounds according to claims 1 to 7, characterized in that in a manner known per se one reacts 3,6-dichloropyridazine having the formula II

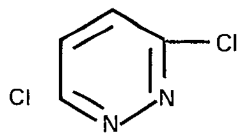

(II)

with isopropylideneglycerol having the formula III

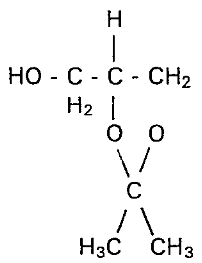

(III)

in the presence of a strong base at temperatures of from 30 to 80°C in an aprotic apolar solvent to 3-chloro-6-(2',2'-dimethyl-1',3'-dioxolan-4'-ylmethoxy)-pyridazine having the formula IV

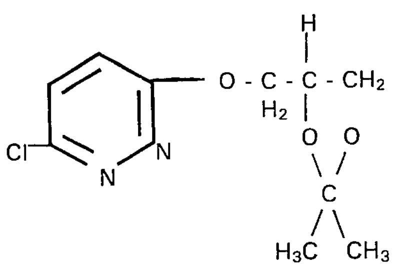

(IV)

one treats the latter with hydrazine at temperatures of from 60 to 90°C and subsequently with acetone at room temperature to 60°C, in each case in protic solvents and one acidifies the reaction mixture at an elevated temperature, one reacts the thus obtained diols 3-(2',3'-dihydroxy-n-prop-1'-oxy)-6-isopropylidenehydrazinopyridazines having the general formula V

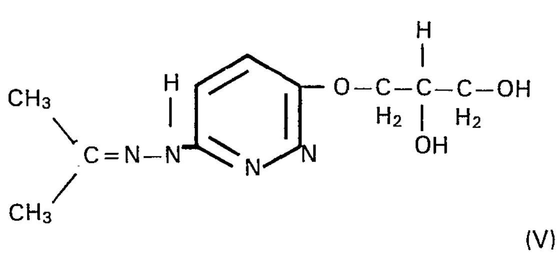

(V)

with orthocarbonic acid alkylesters having the general formula VI

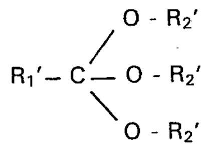

(VI)

wherein $R_1'$ represents hydrogen, an alkyl radical having from 1 to 3 carbon atoms or a phenyl radical and $R_2'$ means an alkyl radical having from 1 to 3 carbon atoms or a phenyl radical in aprotic solvents at temperatures of from 60 to 110°C to 3-(2'-alkoxydioxolan-4'-ylmethoxy)-6-isopropylidenehydrazinopyridazines, optionally 2'-alkylsubstituted at the dioxolane ring, having the general formula VII

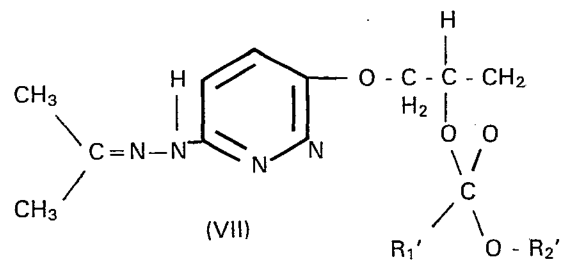

(VII)

wherein $R_1'$ and $R_2'$ are as above defined, one reacts the latter ones with trialkylsilylhalides having the general formula VIII

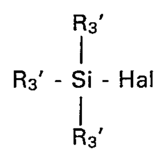

(VIII)

wherein $R_3'$ represents an alkyl radical having from 1 to 4 carbon atoms and Hal means chlorine, bromine or iodine at temperatures of from 20 to 50°C in aprotic solvents containing chlorine to 3-(2'-acyloxy-3'-halogen-n-prop-1'-oxy)-6-isopropylidenehydrazinopyridazines having the general formula IX

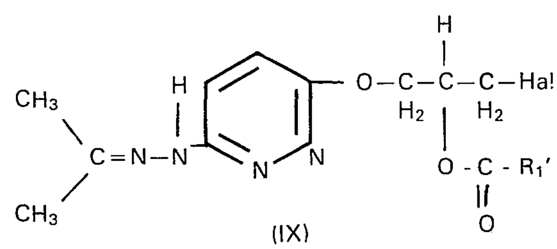

(IX)

wherein $R_1'$ and Hal are as above defined and one reacts the latter ones with amines having the general formula X

$$H_2N - R \qquad (X)$$

wherein R is as defined in claim 1 in protic solvents in the presence of alkali hydroxides, whereupon in a manner known per se optionally one converts the obtained 3-alkoxy-6-isopropylidenehydrazinopyridazines having the general formula I with acids into acid addition salts or optionally one converts the obtained acid addition salts of the 3-alkoxy-6-isopropylidenehydrazinopyridazines having the general formula I into the free 3-alkoxy-6-isopropylidenehy-

drazinopyridazines having the general formula I or into other acid addition salts, respectively, and/or one separates the obtained mixtures of the optically active isomers of the 3-alkoxy-6-isopropylidenehydrazinopyridazines having the general formula I into the optically active isomers or one racemises the obtained optically active isomers of the 3-alkoxy-6-isopropylidenehydrazinopyridazines having the general formula I, respectively.

9. Medicaments, characterized by a content of one or more compounds according to claims 1 to 7 as an active principal, optionally together with 1 or more usual pharmaceutical confectioning agents.

**Claims for the Contracting State:** AT

1. A process for preparing 3-(3'-amino-2'-hydroxy-n-prop-1'-oxy)-6-isopropylidenehydrazinopyridazines having the general formula I

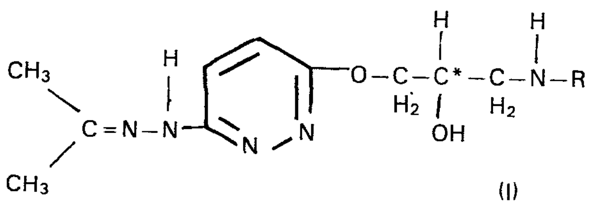

(I)

wherein

R represents an alkyl radical having from 1 to 4 carbon atoms which optionally is substituted by a phenyl radical which moreover may bear from 1 to 3 methoxy radicals and

the asterisk (*) means an asymmetry centre of the molecule

in the form of separated optically active isomers or of mixtures of them as well as their salts with pharmaceutically usable acids, characterized in that in a manner known per se one reacts 3,6-dichloropyridazine having the formula II

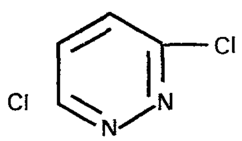

(II)

with isopropylideneglycerol having the formula III

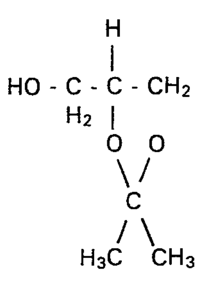

(III)

in the presence of a strong base at temperatures of from 30 to 80°C in an aprotic apolar solvent to 3-chloro-6-(2',2'-dimethyl-1',3'-dioxolan-4'-ylmethoxy)-pyridazine having the formula IV

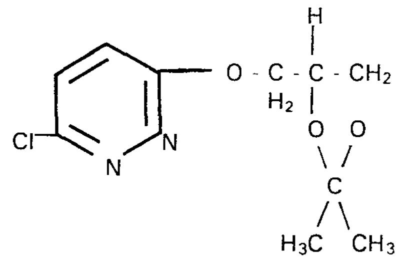

(IV)

one treats the latter with hydrazine at temperatures of from 60 to 90°C and subsequently with acetone at room temperature to 60°C, in each case in protic solvents and one acidifies the reaction mixture at an elevated temperature, one reacts the thus obtained diols 3-(2',3'-dihydroxy-n-prop-1'-oxy)-6-isopropylidenehydrazinopyridazines having the general formula V

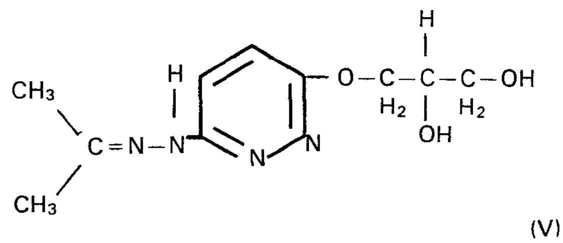

(V)

with orthocarbonic acid alkylesters having the general formula VI

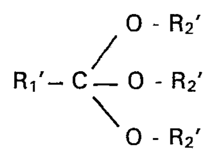

(VI)

wherein $R_1'$ represents hydrogen, an alkyl radical having from 1 to 3 carbon atoms or a phenyl radical and $R_2'$ means an alkyl radical having from 1 to 3 carbon atoms or a phenyl radical in aprotic solvents at temperatures of from 60 to 110°C to 3-(2'-alkoxydioxolan-4'-ylmethoxy)-6-isopropylidenehydrazinopyridazines, optionally 2'-alkylsubstituted at the dioxolane ring, having the general formula VII

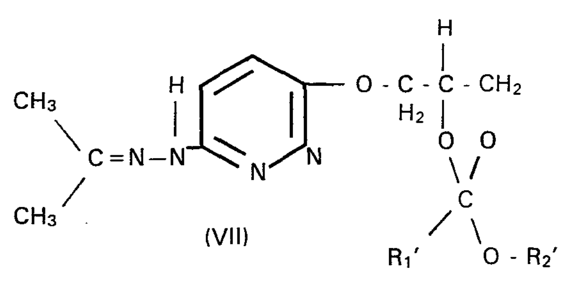

(VII)

wherein $R_1'$ and $R_2'$ are as above defined, one reacts the latter ones with trialkylsilylhalides having the general formula VIII

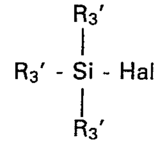

(VIII)

wherein $R_3'$ represents an alkyl radical having from 1 to 4 carbon atoms and Hal means chlorine, bromine or iodine at temperatures of from 20 to 50 °C in aprotic solvents containing chlorine to 3-(2'-acyloxy-3'-halogen-n-prop-1'-oxy)-6-isopropylidenehydrazinopyridazines having the general formula IX

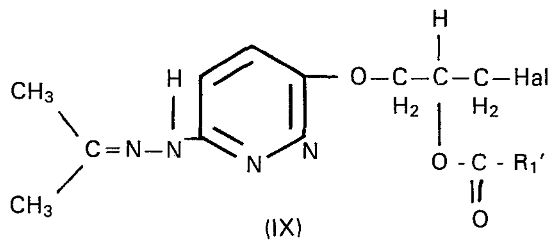

(IX)

wherein $R_1'$ and Hal are as above defined and one reacts the latter ones with amines having the general formula X

$$H_2N - R \qquad (X)$$

wherein R is as defined in claim 1 in protic solvents in the presence of alkali hydroxides, whereupon in a manner known per se optionally one converts the obtained 3-alkoxy-6-isopropylidenehydrazinopyridazines having the general formula I with acids into acid addition salts or optionally one converts the obtained acid addition salts of the 3-alkoxy-6-isopropylidenehydrazinopyridazines having the general formula I into the free 3-alkoxy-6-isopropylidenehydrazinopyridazines having the general formula I or into other acid addition salts, respectively, and/or one separates the obtained mixtures of the optically active isomers of the 3-alkoxy-6-isopropylidenehydrazinopyridazines having the general formula I into the optically active isomers or one racemises the obtained optically active isomers of the 3-alkoxy-6-isopropylidenehydrazinopyridazines having the general formula I, respectively.

2. A process according to claim 1, characterized in that one prepares as salts of the 3-(3'-amino-2'-hydroxy-n-prop-1'-oxy)-6-isopropylidenehydrazinopyridazines of general formula I dihydrochlorides.

3. A process according to claim 1 or 2, characterized in that one prepares 3-(3'-tert.-butylamino-2'-hydroxy-n-prop-1'-oxy)-6-isopropylidenehydrazinopyridazine, 3-(3'-isopropylamino-2'-hydroxy-n-prop-1'-oxy)-6-isopropylidenehydrazinopyridazine, 3-[3'-(n-but-2''-ylamino)-2'-hydroxy-n-prop-1'-oxy]-6-isopropylidenehydrazinopyridazine, 3-/3'-[2''-(3''',4'''-(dimethoxy)-phenyl-eth-1''-ylamino]-2'-hydroxy-n-prop-1'-oxy/-6-isopropylidenehydrazinopyridazine or 3-[3'-(4''-phenyl-n-but-2''-ylamino)-2'-hydroxy-n-prop-1'-oxy]-6-isopropylidenehydrazinopyridazine as well as their salts.

**Revendications pour les Etats contractants:**
BE, CH, DE, FR, GB, IT, LI, NL

1. 3-(3'-amino-2'-hydroxy-n-prop-1'-oxy)-6-isopropylidène-hydrazinopyridazines qui répondent à la formule générale I:

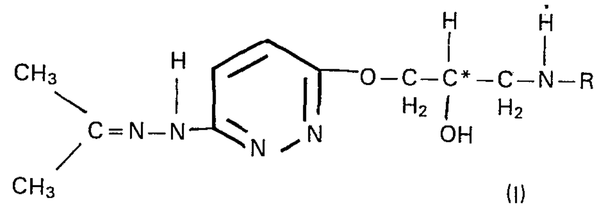

(I)

dans laquelle:

R représente un radical alkyle contenant 1 à 4 atomes de carbone, qui est éventuellement substitué par un radical phényle qui peut encore porter 1 à 3 radicaux méthoxy, et

l'astérique (*) signifie l'existence d'un centre d'asymétrie de la molécule, sous la forme des isomères optiquement actifs séparés ou de mélanges de ceux-ci, ainsi que leurs sels d'acides utilisables du point de vue pharmaceutique.

2. La 3-(3'-tertiobutylamino-2'-hydroxy-n-prop-1'-oxy)-6-isopropylidène-hydrazinopyridazine ainsi que ses sels d'acides utilisables pharmaceutiquement.

3. La 3-(3'-isopropylamino-2'-hydroxy-n-prop-1'-oxy)-6-isopropylidène-hydrazinopyridazine ainsi que ses sels d'acides utilisables pharmaceutiquement.

4. La 3-[3'-(n-but-2''-ylamino)-2'-hydroxy-n-prop-1'-oxy]-6-isopropylidène-hydrazinopyridazine ainsi que ses sels d'acides utilisables pharmaceutiquement.

5. La 3-/3'-[2''-(3''',4'''-(diméthoxy)-phényl)-éth-1''-ylamino]-2'-hydroxy-n-prop-1'-oxy/-6-isopropylidène-hydrazinopyridazine ainsi que ses sels d'acides utilisables pharmaceutiquement.

6. La 3-[3'-(4''-phényl-n-but-2''-ylamino)-2'-hydroxy-n-prop-1'-oxy]-6-isopropylidène-hydrazino pyridazine ainsi que ses sels d'acides utilisables pharmaceutiquement.

7. Sels des 3-(3'-amino-2'-hydroxy-n-prop-1'-oxy)-6-isopropylidène-hydrazinopyridazines selon les revendications 1 à 6, caractérisés par le fait qu'ils sont des dichlorhydrates.

8. Procédé de préparation des composés selon les revendications 1 à 7, caractérisé en ce que de manière connue en elle-même, on fait réagir la 3,6-dichloropyridazine de formule II:

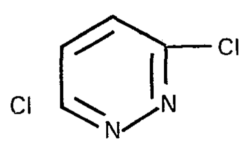

(II)

avec l'isopropylidèneglycérol de formule III:

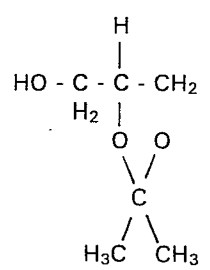

(III)

en présence d'une base forte, à des températures comprises entre 30 et 80 °C, dans un solvant apolaire aprotonique, pour obtenir la 3-chloro-6-(2',2'-diméthyl-1',3'-dioxolan-4'-ylméthoxy)-pyridazine de formule IV:

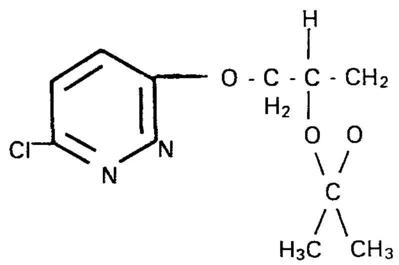

(IV)

en ce que l'on traite cette dernière par de l'hydrazine à des températures comprises entre 60 et 90 °C et ensuite par l'acétone à température comprise entre la température ambiente et 60 °C, chaque fois dans des solvants protoniques et en ce que l'on acidifie le mélange réactionnel à température élevée, en ce que l'on fait réagir les diols obtenus qui sont des 3-(2',3'-dihydroxy-n-prop-1'-oxy)-6-isopropylidène-hydrazinopyridazines de formule générale V:

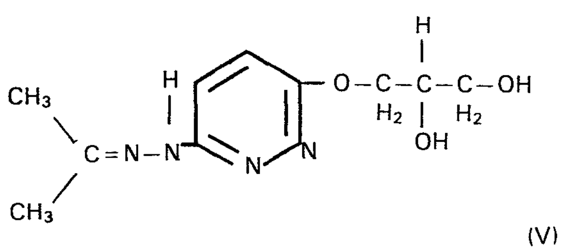

(V)

avec des alkylesters d'acides orthocarboxyliques de formule générale VI:

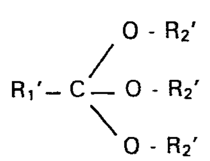

(VI)

dans laquelle $R_1'$ représente l'hydrogène, un radical alkyle contenant 1 à 3 atomes de carbone ou un radical phényle et $R_2'$ représente un radical alkyle contenant 1 à 3 atomes de carbone ou un radical phényle, dans des solvants aprotoniques, à des températures comprises entre 60 et 110 °C, pour obtenir des 3-(2'-alcoxydioxolan-4'-ylméthoxy)-6-isopropylidène-hydrazinopyridazines portant éventuellement un substituant 2'-alkyle sur le noyau dioxolane, de formule générale VII:

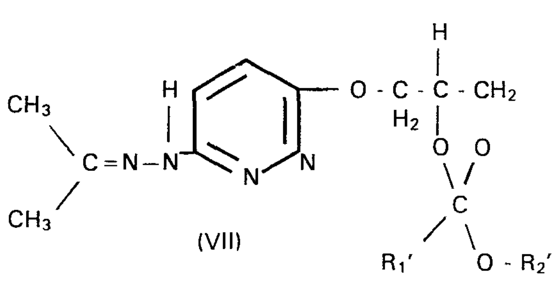

(VII)

dans laquelle $R_1'$ et $R_2'$ sont tels que définis plus haut, en ce que l'on fait réagir ces dernières avec des halogénures de trialkylsilyle de formule générale VIII:

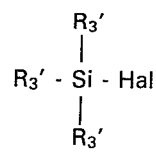

(VIII)

dans laquelle $R_3'$ représente un radical alkyle contenant 1 à 4 atomes de carbone et Hal représente le chlore, le brome ou l'iode, à des températures comprises entre 20 et 50 °C, dans des solvants aprotoniques contenant du chlore, pour obtenir des 3-(2'-acyloxy-3'-halogéno-n-prop-1'-oxy)-6-isopropylidène-hydrazinopyridazines de formule générale IX:

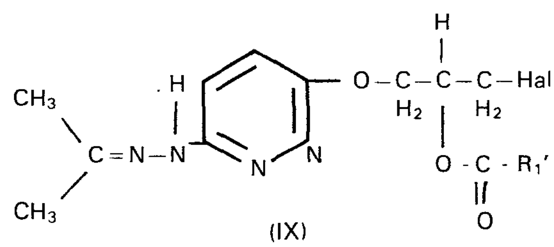

(IX)

dans laquelle $R_1'$ et Hal sont tels que définis plus haut et en ce qu'on fait réagir ces dernières avec des amines de la formule générale X:

$$H_2N - R \qquad (X)$$

dans laquelle R est tel que défini dans la revendication 1, dans des solvants protoniques, en présence d'hydroxydes alcalins, après quoi, de manière en elle-même connue, on convertit éventuellement les 3-alcoxy-6-isopropylidène-hydrazinopyridazines de formule I obtenues, avec des acides, en sels d'addition d'acide ou éventuellement on convertit les sels d'addition d'acide des 3-alcoxy-6-isopropylidène-hydrazinopyridazines de formule générale I obtenus en 3-alcoxy-6-isopropylidène-hydrazinopyridazines libres de formule générale I ou en d'autres sels d'addition d'acide et/ou éventuellement on dédouble les mélanges d'isomères optiquement actifs des 3-alcoxy-6-isopropylidène-hydrazinopyridazines de formule générale I obtenus pour obtenir les isomères optiquement actifs, ou bien on racémise les isomères optiquement actifs des 3-alcoxy-6-isopropylidène-hydrazinopyridazine de formule générale I obtenus.

9. Médicaments caractérisés par une teneur en un ou plusieurs composés selon les revendications 1 à 7 comme constituant(s) actif(s), éventuellement en même temps qu'un ou plusieurs agents de conditionnement pharmaceutiques usuels.

**Revendications pour l'Etat contractant:** AT

1. Procédé de préparation de 3-(3'-amino-2'-hydroxy-n-prop-1'-oxy)-6-isopropylidène-hydrazinopyridazines qui répondent à la formule générale I:

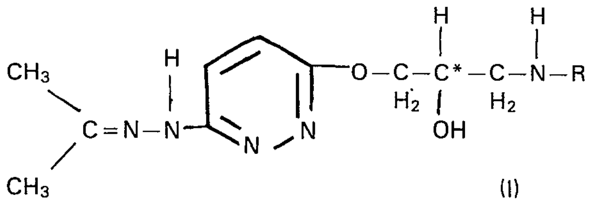

(I)

dans laquelle:

R représente un radical alkyle contenant 1 à 4 atomes de carbone, qui est éventuellement substitué par un radical phényle qui peut encore porter 1 à 3 radicaux méthoxy, et

l'astérique (*) signifie l'existence d'un centre d'asymétrie de la molécule, sour la forme des isomères optiquement actifs séparés ou de mélanges de ceux-ci, ainsi que leurs sels d'acides utilisables du point de vue pharmaceutique, caractérisé en ce que de manière connue en elle-même, on fait réagir la 3,6-dichloropyridazine de formule II:

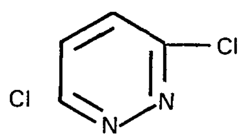

(II)

avec l'isopropylidèneglycérol de formule III:

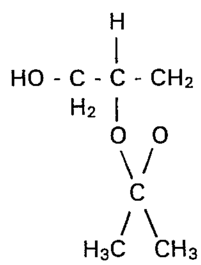

(III)

en présence d'une base forte, à des températures comprises entre 30 et 80°C, dans un solvant apolaire aprotonique, pour obtenir la 3-chloro-6-(2',2'-diméthyl-1',3'-dioxolan-4'-ylméthoxy)-pyridazine de formule IV:

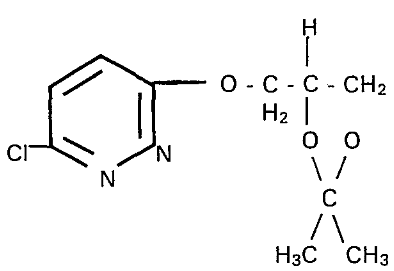

(IV)

en ce que l'on traite cette dernière par de l'hydrazine à des températures comprises entre 60 et 90°C et ensuite par l'acétone à une température comprise entre la température ambiente et 60°C, chaque fois dans des solvants protoniques et en ce que l'on acidifie le mélange réactionnel à température élevée, en ce que l'on fait réagir les diols obtenus qui sont des 3-(2',3'-dihydroxy-n-prop-1'-oxy)-6-isopropylidène-hydrazinopyridazines de formule générale V:

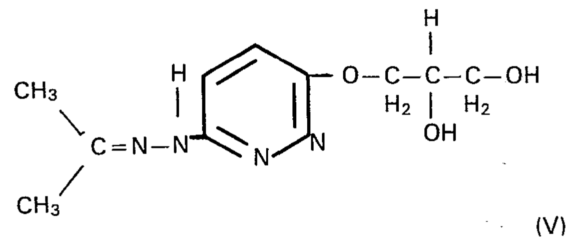

(V)

avec des alkylesters d'acides orthocarboxyliques de formule générale VI:

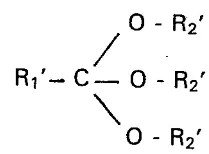

(VI)

dans laquelle $R_1'$ représente l'hydrogène, un radical alkyle contenant 1 à 3 atomes de carbone ou un radical phényle et $R_2'$ représente un radical alkyle contenant 1 à 3 atomes de carbone ou un radical phényle, dans des solvants aprotoniques, à des températures comprises entre 60 et 110°C, pour obtenir des 3-(2'-alcoxydioxolan-4'-ylméthoxy-6-isopropylidène-hydrazinopyridazines portant éventuellement un substituant 2'-alkyle sur le noyau dioxolane, de formule générale VII:

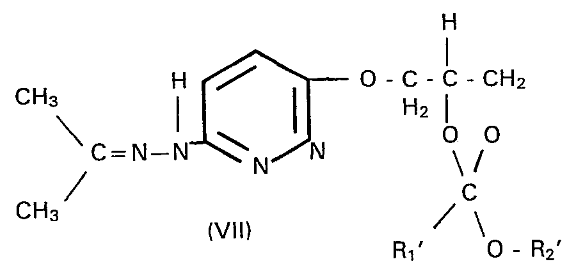

(VII)

dans laquelle $R_1'$ et $R_2'$ sont tels que définis plus haut, en ce que l'on fait réagir ces dernières avec des halogénures de trialkylsilyle de formule générale VIII:

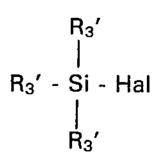

(VIII)

dans laquelle $R_3'$ représente un radical alkyle contenant 1 à 4 atomes de carbone et Hal représente le chlore, le brome ou l'iode, à des températures comprises entre 20 et 50°C, dans des solvants aprotoniques contenant du chlore, pour obtenir des 3-(2'-acyloxy-3'-halogéno-n-prop-1'-oxy)-6-isopropylidène-hydrazinopyridazines de formule générale IX:

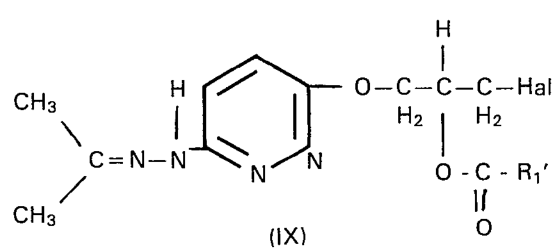

(IX)

dans laquelle $R_1'$ et Hal sont tels que définis plus haut et en ce qu'on fait réagir ces dernières avec des amines de la formule générale X:

$$H_2N - R \qquad (X)$$

dans laquelle R est tel que défini dans la revendication 1, dans des solvants protoniques, en présence d'hydroxydes alcalins, après quoi, de manière en elle-même connue, on convertit éventuellement les 3-alcoxy-6-isopropylidène-hydrazinopyridazines de formule I obtenues, avec des acides, en sels d'addition d'acide ou éventuellement on convertit les sels d'addition d'acide des 3-alcoxy-6-isopropylidène-hydrazinopyridazines de formule générale I obtenus en 3-alcoxy-6-isopropylidène-hydrazinopyridazines libres de formule générale I ou en d'autres sels d'addition d'acide et/ou éventuellement on dédouble les mélanges d'isomères optiquement actifs des 3-alcoxy-6-isopropylidène-hydrazinopyridazines de formule générale I obtenus pour obtenir les isomères optiquement actifs, ou bien on racémise les isomères optiquement actifs des 3-alcoxy-6-isopropylidène-hydrazinopyridazine de formule générale I obtenus.

2. Procédé de préparation selon la revendication 1, caractérisé en ce qu'on prépare comme sels des 3-(3'-amino-2'-hydroxy-n-prop-1'-oxy)-6-isopropylidène-hydrazinopyridazines de formule générale I dichlorhydrates. 3. Procédé de préparation selon la revendication 1 ou 2, caractérisé en ce qu'on prépare 3-(3'-tertiobutylamino-2'-hydroxy-n-prop-1'-oxy)-6-isopropylidène-hydrazinopyridazine, 3-(3'-isopropylamino-2'-hydroxy-n-prop-1'-oxy)-6-isopropylidène-hydrazinopyridazine, 3-[3'-(n-but-2''-ylamino)-2'-hydroxy-n-prop-1'-oxy]-6-isopropylidène-hydrazinopyridazine, 3- /3'-[2''-(3''',4'''-/diméthoxy/-phényl)-éth-1''-ylamino]-2'-hydroxy-n-prop-1'-oxy/-6-isopropylidène-hydrazinopyridazine ou 3-[3'-(4''-phényl-n-but-2''-ylamino)-2'-hydroxy-n-prop-1'-oxy]-6-isopropylidène-hydrazinopyridazine ainsi que leurs sels.